# EUROPEAN PATENT APPLICATION

(11) **EP 2 599 477 A1**
(43) Date of publication of application: **05.06.2013**
(21) Application number: 11009477.8
(22) Date of filing: 30.11.2011
(51) Int. Cl.: A61K 9/16, A61K 9/20, A61K 9/50, A61K 31/192, A61K 9/00

(54) **4-Phenylbutyric acid sustained release formulation**

(71) Applicant: Lunamed AG, 7000 Chur (CH)
(72) Inventor: The designation of the inventor has not yet been filed
(74) Representative: Peters, Hajo

(57) **Abstract**

The present invention refers to a sustained release formulation for 4-phenylbutyric acid which comprises pellets containing 4-PBA, wherein the 4-PBA is in the form of the free acid. The invention relates further to the preparation of the pellet formulation and their therapeutical use.

## Description

### Field of the invention

The present invention refers to relates to a pharmaceutical composition for the sustained release of 4-phenylbutyric acid (4-PBA). Said pharmaceutical composition includes pellets comprising 4-phenylbutyric acid in the form of the free acid. The invention relates further to the preparation and the therapeutic use of said pharmaceutical pellet formulation.

### Background of the invention

Phenylbutyric acid is a well known compound to which a number of medical uses have been ascribed in patent or scientific literature. In form of its sodium salt it is marketed as a drug in the USA and European Union. Sodium phenylbutyrate is an orphan drug, marketed by Ucyclyd Pharma (Hunt Valley, USA) under the trade name Buphenyl and by Swedish Orphan International (Sweden) as Ammonaps for the treatment of urea cycle disorders.

Over the years a number of salts of phenylbutyric acid has been described. Still, except for sodium phenyl butyrate none of them has - for various reasons - reached the market. On the other hand the marketed sodium phenylbutyrate can be - especially in the indication marketed for but also in other circumstances - quite disadvantageous. According to Summary of the Product Characteristics (ANNEX I published by the EMEA) under 4.4 (Special Warnings) it is stated:
*"Each AMMONAPS tablet contains 62 mg (2.7 mmol) of sodium, corresponding to 2.5 g (108 mmol) of sodium per 20 g of sodium phenylbutyrate, which is the maximum daily dose. AMMONAPS should therefore be used with caution in patients with congestive heart failure or severe renal insufficiency, and in clinical conditions where there is sodium retention with oedema."*

In addition, it was further intended to identify a form of the active ingredient that would allow a favorable use in a slow-release formulation.

The international application WO2006/059237 discloses a 4-PBA containing controlled release formulation, whereby the 4-PBA is dispersed in a polymer matrix.

Furthermore, the European application EP 1 427 396 A1 discloses a prolonged release form of sodium 4-phenylbutyrate comprising 2-25% of a release-controlling excipient.

The US patent application US2004/0152784 described a dry granulated pharmaceutical composition of sodium phenylbutyrate that can be dissolved in water before administration. Hence, it was the goal of this invention to provide an alternative pharmaceutical composition containing 4-phenylbutyric acid which overcomes at least some of the above described disadvantages of prior art formulations.

It has now been found that a pellet formulation comprising phenylbutyric acid in the form of the free acid allows a sustained release of the drug and furthermore represents a sodium-free formulation.

Accordingly, the invention is drawn to a pellet formulation comprising 4-phenylbutyric acid, wherein the 4-PBA is in the form of the free acid.

Due to the low solubility of the free acid of 4-PBA, the 4-PBA is slowly absorbed within the gastro-intestinal tract, which enable a sustained release of 4-PBA.

In addition, due to the lack of sodium, the 4-PBA formulation according to the invention can be also given to patients with congestive heart failure, severe renal insufficiency or sodium retention with oedema.

Furthermore, as a pellet composition the sustained release formulation of the invention exhibit several advantages:
□ The use of the sparsely soluble free acid allows a sustained release without the need for further release-modifying excipients.
□ The pelletization offers flexibility in dosage form design and development.
□ Pellets are less susceptible to dose dumping.
□ Pellets offer reduced variation in gastric emptying rate and transit time.
□ Pellets disperse freely in G.I.T. and invariably maximize drug absorption and also reduce peak plasma fluctuation.
□ Pellets ensure improved flow properties in formulation development
□ The improved appearance of the product and the core is pharmaceutically elegant.
□ The smooth surface & the uniform size of the pellets allow uniform coating not only for each pellet but also from batch to batch.
□ Pellets open up the opportunity that chemically incompatible products can be formed into pellets and delivered in a single dose by encapsulating them.
□ The generation of powder dusting within the production process is avoided.
□ The beads or granules of different thickness of coatings are blended in the desired proportions to give the desired effect.
□ The rate at which the 4-PBA is released from the coated particle can be controlled by the thickness of the coat on the pellets.
□ The pellets according to the invention possess a size between 0,2 mm and 2 mm can pass the pylorus although the sphincter is closed. As a consequence, their transit rate is independent of the filling level of the stomach.

By sustained release of the active ingredient is to be understood especially a rate of release of the active ingredient during a period of about 6 to 12 or up to 24 hours.

A "pellet" according to the invention is defined as a spherical, rounded, ovoid or cylindrical particle with a size between 0.2 and 2 mm. Preferably, they are isometric aggregates with smooth surfaces and narrow particle size distribution.

A "pharmaceutical composition" is defined as any composition, other than food, used in the prevention, diagnosis, alleviation, treatment, or cure of disease in man and animal.

According to the invention the free acid form of 4-PBA includes also a solvate or a hydrate of the free acid.

"Hydrate" is defined as a substance/joint mixture according to the invention containing water. Thus, it may be a crystalline salt with one or more water molecules in the crystalline structure or also amorphous containing water molecules.

"Solvate" is defined as a substance/joint mixture according to the invention containing a further solvent. One most prominent example of the solvents is an alcohol like ethanol or methanol thus leading to methanolates or ethanolates. Thus, it may be a crystalline salt with one or more alcohol molecules in the crystalline structure or also an amorphous form containing alcohol molecules.

In a broader aspect of the invention, the invention is also drawn to any sustained release formulation comprising 4-phenylbutyric acid, wherein the 4-PBA is in the form of the free acid. In one embodiment of this broader aspect, the proviso applies that the 4-PBA is not dispersed in a polymer matrix.

### Detailed description of the invention

In a first aspect the invention relates to a sustained release formulation comprising pellets which comprise the free acid form of 4-phenylbutyric acid (4-PBA).

In a second aspect of the invention the sustained release formulation according to the invention comprises a homogenous pellet.

As defined herein, a homogenous pellet is homogenously composed of the active ingredient or a mixture of the active ingredient with at least one other compound, being preferably a matrix forming polymer. The active compound is preferably homogeneously dispersed in the matrix described above and is released with a delay after dissolving the enteric coating. The matrix with the active compound homogeneously dispersed therein advantageously extends through the entire core. Homogenous pellets are predominantly produced by extrusion or spheronisation/marumerization.

In a further aspect of the invention, the sustained release formulation according to the invention comprises a heterogeneous pellet. Heterogeneous pellets consist of a core region and at least one coating region. Heterogeneous pellets develop through coating of the core region.

In one embodiment of the invention the core region of the heterogeneous pellet is build from an inert core which does not contain the active ingredient. This starter core is predominantly made from a starch or a sugar derivative or a mixture thereof.

In a further embodiment of the invention the inert core is a Non-Pareil seeds or micro globule. These Non-Pareil Seeds or micro globules are small spherical granules made from Pharma Grade Sugar in sizes ranging from 420 - 1400 microns.

The active ingredient 4-PBA is used in the form of its free acid. Either the 4-PBA is used as starter cores in the form of coarse crystals, preferably in the particle size range 0.2 - 0.5 mm or 0.4 — 1 mm, or the 4-PBA in the form of fine crystals (e.g. <50 micrometers or <10 micrometers) or coarse crystals (e. g. <250 micrometers), in a suspension with adjuncts, is applied to starter cores, e.g. consisting of sugar (sucrose), which optionally contain additives such as sodium a carboxymethyl starch, polyvinylpyrrolidone, gelatine or other compounds known to those skilled in the art.

The suspension or solution applied to the starter cores contains 4-PBA and adjuncts, especially at least one binder, at least one sedimentation retarder, pH correctors and optionally at least one pigment and/or lacquers, lubricants/antiadhesive agents and suspension stabilizers/thickeners in water or a mixture of water and one or more conventional organic solvents, or in an organic solvent.

Examples of suitable binders are sodium carboxymethyl starch, polyvinylpyrrolidone, gelatine, hydroxypropylmethyl cellulose, hydroxyethyl cellulose, xanthan, carrageenan products, polyvinyl acetate, sodium carboxymethyl cellulose, ethyl cellulose, starch mucilage or liquefied waxes, either individually or in combination with one another.

Examples of suitable sedimentation retarders are highly disperse silicon dioxide, starch mucilage, mucilages like tragacanth, gums, e. g. gum arabic, xanthans, aliginates or carrageenan products, either on their own or in combination with one another.

Examples of suitable pH correctors are sodium hydroxide, hydrochloric acid, methylglucamine or buffer substances like sodium dihydrogen phosphate or disodium hydrogen phosphate.

Examples of pigments or lacquers which can be used are iron oxides, erythrosine, yellow orange S, tartraziane, phthalocyanine blue or indigotin.

Talcum is a particularly suitable lubricant/antiadhesive agent and highly disperse silicon dioxide is a particularly suitable suspension stabilizer/thickener.

One optional excipient that may be used to prepare the core is a surfactant. If a surfactant is employed, it can be any type of surfactant commonly known in the art such as a fatty acid, a chelating agent, a bile salt or mixtures thereof. Examples of some preferred surfactants are fatty acids such as capric acid, oleic acid and their monoglycerides, especially alkyl sulfates, such as sodium lauryl sulfate, sodium dodecyl sulfate and polysorbate 80; chelating agents such as citric acid and phytic acid. The preferred surfactant used herein is sodium lauryl sulphate.

The core of a pharmaceutical formulation according to the invention contains 4-PBA in an amount of 20 - 70% by weight, preferably 30 - 50% by weight, and adjuncts in an amount of 80 to 30% by weight, preferably 70 - 50% by weight, based in each case on the core of the pellet and deducting the weight of the starter cores.

### Control release coating

In a further embodiment of the invention the pellet is coated by a control release membrane. This membrane is applied to the core once the core is prepared. The controlled release membrane is applied so that it prevents or retards the release of the drug from the core. The controlled release coat is preferably comprised of a combination of polymeric film forming polymers and may optionally contain conventional processing aids such as emulsifiers, plasticizers, surfactants, lubricants or channeling agents.

The film forming polymers suitable for use in the controlled release coating are the water insoluble polymeric coating agents such as such as, ethylcellulose, cellulose acetate, cellulose propionate (lower, medium or higher molecular weight), cellulose acetate propionate, cellulose acetate butyrate, triacetate, cellulose tricetate, poly(methyl methacrylate), poly(ethyl methacrylate), poly(butyl methacrylate), poly(isobutyl methacrylate), poly(hexyl methacrylate), poly(isodecyl methacrylate), poly(lauryl methacrylate), poly(phenyl methacrylate), poly(methyl acrylate), poly(isopropyl acrylate), poly(isobutyl acrylate), poly(octadecyl acrylate), poly(ethylene acrylate), poly(ethylene), poly(ethylene) low density, poly(ethylene) high density, poly(propylene), poly(ethylene terephthalate), poly(vinyl isobutyl ether), poly(vinyl acetate), poly(vinyl chloride), chitin, chitosan, poly(anhydrides), poly(lactic acid), ploy(glycolic acid), poly(ortho esters), poly(lactide co-glycollide), poly(hydroxy butyrate) or polyurethane or a mixture thereof.

The controlled release coating should also contain a pore forming agent, which is a material that will dissolve or hydrate in gastrointestinal fluid and create channels or pores to aid in the release of the drug. Some common pore forming agents are water soluble materials such as sugars (i.e., sucrose, lactose fructose) and salts. In one embodiment of the present invention the controlled release membrane employs a pH dependent pore forming agent. A pH dependent pore forming agent is a material that dissolves only at certain pHs. An example of a suitable pH dependent pore forming agent is an enteric polymer. Enteric polymers are well known in the art and any suitable enteric polymers may be used. Preferably, the enteric polymer is selected from cellulose acetate phthalate, hydroxypropyl methylcellulose phthalate, polyvinylacetate phthalate, methacrylic acid copolymer, shellac, hydroxypropyl methylcellulose succinate, cellulose acetate trimellitate, hypromellose phthalate and mixtures of any of the foregoing. The preferred pore forming agent is hypromellose phthalate.

The controlled release coating may be built up by applying a plurality of coats of polymer solution or suspension to the drug core as hereinafter described. The membrane solution or suspension contains the polymer(s) dissolved or suspended, respectively, in a suitable aqueous or organic solvent or mixture of solvents, optionally in the presence of other conventional excipients.

The controlled release coating solution or suspension may be applied to the active cores in a conventional coating pan as indicated or, alternatively, using an automated system such as a CF granulator, for example a FREUND CF® granulator, a GLATT® fluidized bed processor, a modified ACCELA-COTA® or other suitably automated bead coating equipment.

Suitable emulsifiers that can be used in the present invention may include, but are not limited to, phospholipids, polysorbate, propylene glycol, poloxamer, glyceryl monostearate, other pharmaceutical emulsifiers and/or mixtures thereof.

Suitable surfactants that may optionally be used in the present invention are sodium lauryl sulfate, sodium taurocholate or a polysorbate.

The controlled release coating may optionally include a plasticizing agent. Plasticizers are used to increase the resiliency of the finished product from cracking and fracturing. Suitable plasticizing agents include polyethylene glycol, propylene glycol, glycerol, triacetin, dimethyl phthalate, diethyl phthalate, dibutyl phthalate, dibutyl sebacate, triethyl citrate, acetyltributyl citrate, tributyl citrate, triethyl acetyl citrate, castor oil, poloxamers and varying percentages of acetylated monoglycerides. The preferred plasticizer is acetyltributyl citrate.

Suitable lubricants possess anti-sticking or anti-tacking properties. Suitable lubricants used in preparing solid dosage forms may include talc, stearic acid, magnesium stearate, glyceryl monostearate, sodium stearyl fumerate, hydrogenated oils, polyethylene glycols and sodium stearate. A particularly preferred lubricant is talc.

### Neutral film membrane

In another embodiment of the invention the pellet core is coated by a neutral film membrane e.g. a coating having no retarding action. Such a neutral film membrane consists of fast-dissolving constituents. In this case it is preferable to use low-viscosity hydroxypropylmethylcellulose tye 2910 USP XXII, for example Methocel ® E5 or E 15 (Dow Chemicals Ltd.) or Pharmacoat ® 606 (Shin Etsu).

### Enteric coating

Medicaments or pharmaceutical formulation according to the present invention may also comprise an enteric coating, so that their dissolution is dependent on pH-value. Due to said coating the medicament may pass the stomach undissolved and the respective salts or their respective crystalline form is liberated in the intestinal tract. Preferably the enteric coating is soluble at a pH value of 5 to 7.5. Suitable materials and methods for the preparation are known from the prior art. (e. g. EP 0 453 001 A1).

Preferred enteric coatings according to the invention comprise a methacrylic acid copolymer or methylhydroxypropylcellulose phthalate. Poly(methacrylic acid, methyl methacrylates), which are obtainable under the tradenames Eudragit L or S and have free carboxyl groups as functional groups, are preferred. These polymers are insoluble in the gastric juice, but dissolve in digestive juices above pH 5. 5-7 depending on the number of functional carboxyl groups. Poly(methacrylic acid, methyl methacrylate) 1:1 (Eudragit L 100; methacrylic acid copolymer, USP/NF type A) and poly(methacrylic acid, methyl methacrylate) 1:2 (Eudragit S; methacrylic acid copolymer, USP/NF type B) are particularly preferred. Eudragit L 100 is the most preferred. Mixtures of the coating materials mentioned, in particular of Eudragit L and Eudragit S, can also be used.

The thickness of the enteric coating may vary widely without influencing the in vitro release of 4-PBA in test solutions which mimic in vivo conditions in man. To prevent the 4-PBA release in the stomach, the enteric coating constitutes at least an amount of 1.0 % by weight of the core weight, preferably at least 3.0 % and especially at 30 least 6.0 %. The upper amount of the applied enteric coating is normally only limited by processing conditions. This possibility to vary the thickness of the enteric coating without deleterious influence on the release of 4-PBA is especially desirable in large scale processes. The enteric coating layer(s) may be applied on the pre-processed formulation containing subcoating layer(s) without exactly controlling the thickness of the applied coating layer(s).

The pellet formulation can comprise one or more coatings, however pellet formulations in which the pellet only comprises one coating are preferred.

### Processing to a final dosage form

In a further embodiment of the invention the pellets can be processed to a final dosage form is either an (preferably enteric coated) tablet or capsule or in the case of enteric coated pellets or granules, these pellets or granules dispensed in hard gelatin capsules or sachets. The final dosage form may further be coated with an additional layer containing pigment(s) and/or colorant(s).

During tabletting, the pellet formulation or granule formulation is converted into a compressed tablet by applying a high pressure, if appropriate in the presence of free active compound and other additives (initial dose). By combination with corresponding auxiliaries, almost any active compound can be converted into a tablet which, in addition, can also have certain properties necessary for its use.

The galenical properties of tablets, in particular their disintegration in water or in digestive juices and their mechanical characteristics are decided by the amount and the physicochemical properties of the active compounds and auxiliaries, in addition to the production process.

Auxiliaries for the production of tablets are classified into the following most important types:
□ Fillers, for example lactose and sucrose;
□ Binders, for example starch, gelatin, sugar, cellulose ethers and polymers, for example polyvinylpyrrolidone disintegrating agents, for example starch, starch ethers and Kollidon ®CL;
□ Lubricants and mold release agents, for example talc, stearates and silicones;
□ Flow control agents, for example highly disperse silicon dioxide and talc.
□ Lacquers

Capsules can likewise be produced by methods, which are described in "Ullmann, Volume 18, Pharmazeutische Technologie" (Pharmaceutical Technology), using pellet formulations according to the invention.

By varying the binder, the binder concentration in the pellets or granules and the production process, it is possible to control the dissolution time for the pharmaceutical active compound in the small intestine. If, for example, rapid disintegration in the small intestine is desired, it is possible to add, if appropriate, a disintegrating agent, such as Kollidon ®CL, starch, UAP, Avicel ® and the like, or a disintegration accelerator, such as Aerosil ®, surfactants and the like. (Kollidon®CL = crosslinked polyvinylpyrrolidone, UAP = ultra-amylopectin, Avicel ® = microcrystaline cellulose and Aerosil ® = highly disperse SiO₂). The lacquering which is resistant to gastric juice can be applied by the customary lacquering processes, such as, for example, in a fluidized bed or an open or closed kettle system.

To prepare tablets containing the pellet formulations according to the invention, the usual tabletting processes are resorted to (see, for example, Ullmann, Volume 18, Pharmazeutische Technologie (Pharmaceutical Technology)).

### Synthesis of 4-PBA

The 4-PBA as active ingredient of the pellet formulation can be prepared by various ways. As an example, phenylbutyrate derivatives have been prepared by the Arndt-Einstert reaction, using diazomethane with silver oxide and sodium thiosulfate (J. Chern. Soc., 1997-99 (1938)). Alternatively, thianapthene-2-acetic acid and thianapthene-3-acetic acid have been used to prepare p-phenylbutyric acid (J. Am. Chern. Soc., 70, 3768 (1948)). The Gignard reagent, benzyl magnesium chloride, has also been used in the synthesis of phenylbutyric acid, resulting in a yield of 16.1 % (J. Am. Chern. Soc., 71, 2807-2808 (1949)). An improved synthesis strategy for 4-PBA was disclosed by the international application WO 2002/094756 by reacting benzene with butyrolactone in the presence of aluminium chloride, followed by neutralization with base.

The sustained release formulation according to the invention can be of any form suitable for the application to humans and/or animals, preferably humans including infants, children and adults and can be produced by standard procedures known to those skilled in the art. The pharmaceutical composition can be produced by standard procedures known to those skilled in the art, e.g. from the table of contents of "Pharmaceutics: The Science of Dosage Forms", Second Edition, Aulton, M.E. (ED. Churchill Livingstone, Edinburgh (2002); "Encyclopedia of Pharmaceutical Technology", Second Edition, Swarbrick, J. and Boylan J.C. (Eds.), Marcel Dekker, Inc. New York (2002); "Modern Pharmaceutics", Fourth Edition, Banker G.S. and Rhodes C.T. (Eds.) Marcel Dekker, Inc. New York 2002 y "The Theory and Practice of Industrial Pharmacy", Lachman L., Lieberman H. And Kanig J. (Eds.), Lea & Febiger, Philadelphia (1986). The respective descriptions are hereby incorporated by reference and form part of the disclosure. The composition of the medicament may vary depending on the application scheme.

### Pellet manufacturing

A process for the manufacture of a dosage form according to the present invention represents a further aspect of the invention. Several production strategies are known to the person skilled in art. In the following a non-exhaustive list of production methods is disclosed:

### Moist granulation,

In moist granulation, the powder mixture is processed with a liquid to a paste, from which the granules are formed, after drying, evaporation or solidification of the granulation liquid. A differentiation is made between:

### (a) Build-up Granulation

The powder mixture is kept in motion and is brought to the desired granule size by spraying with granulation liquid.

In the plate granulation process, the powder mixture to be granulated is introduced onto a plate rotating at an angle. The granules formed are spherical. In fluidized bed granulation, the powder mixture is whirled up in a stream of air, kept in suspension and sprayed with the granulation liquid. In this case also, substantially spherical granule grains are formed.

### (b) Breaking-down Granulation

The powder mixture is thoroughly uniformly moistened with the granulation liquid by kneading with a kneader and is pressed, in paste consistency, through a sieve or a perforated roll or perforated disc. After drying, the cylindrical or parallelepipedal granules are sieved out, if appropriate, to the desired particle size. The granulation liquid used is, depending on the solubility of the active compound, preferably water, and in addition, for example, water/alcohol mixtures or solutions of binders. Suitable binders are macromolecular substances, such as cellulose derivatives, gelatin, starch, polyvinylpyrrolidone or alginates.

### Melt granulation

Sinter or melt granules are formed by melting constituents of the powder mixture and shaping them through a sieve.

### Dry granulation,

In dry granulation, the powder mixture is shaped into relatively large, coarse pressed pieces, so-called briquettes, in tabletting machines or mills, and are coarsely comminuted and sieved to the desired granule size. The process is particularly used in processing active compounds which are unstable to heat or sensitive to moisture.

In a further aspect of the invention other pelletization methods such as globulation, cryopelletization, balling, compression can be also used:
Globulation or droplet formation consists two related processes, spray drying and spray congealing. During the spray drying process the 4-PBA is sprayed in the suspension or solution without excipients into a hot stream to produce dry and more spherical particles.

Spray congealing is characterized by a process in which the 4-PBA is allowed to melt, disperse or dissolve in hot melts of gums, waxes or fatty acids, and is sprayed into an air chamber where the temperature is kept bellow the melting point of the formulation components, to produce spherical congealed pellets. Controlled release pellets can be prepared in this process depending on the physiochemical properties of the ingredients and other formulation variables.

Cryopelletization is a process in which the liquid formulation is converted in to solid spherical particles or pellets in the presence of liquid nitrogen as fixing medium. The shape depends up on the distance the droplet travel before contacting liquid nitrogen.

Compression is one type of compaction technique for preparing pellets. Compacting mixtures or blends of active ingredients and excipients under pressure prepare pellets of definite sizes and shapes. The formulation and process variables controlling the quality of pellets prepared are similar to those used in tablets manufacturing.

Balling is the pelletization process in which pellets are formed by a continuous rolling and tumbling motion in pans, discs, drums or mixtures. The process consists of conversion of finely divided particles in to spherical particles upon the addition of appropriate amounts of liquid.

In one embodiment of the invention the heterogenous pellets are prepared according to the method disclosed in the international application WO 2004/108266, which is incorporated herein by reference.

A further aspect of the invention refers to the pharmaceutical composition according to the invention for use in the treatment of cancer, mucositis, amyotropic lateral sclerosis (ALS), skin/mucosal itching, degenerative diseases of the eye, neurodegenerative disorders, Huntington's disease, eating disorders, obesity, drug induced weight gain, pruritis, alcoholism, promyelocytic leukemia, especially acute promyelocytic leukemia, cardiac injury, especially adriamycin induced cardiac injury, epithelial damage, connective tissue damage, desmosis, Parkinson Disease, myelodysplastic syndrome, urea cycle disorder, fibrotic lung diseases, hepatic encephalopathies, thioredoxin mediated diseases, human papilloma virus (HPV) infections, autoimmune diseases, thalassemia, genetic disorders; or in the regulation of HDAC6 activity, the modulation of stem cells, the prevention of grey hair, the promotion of neuronal growth, the prevention of bone and joint diseases, or as adjuvant in cancer therapy or in the treatment of Alzheimer's Disease.

### Therapeutic use

A further aspect of the invention refers to the pharmaceutical composition according to the invention (or the use of a pharmaceutical composition according to the invention in the manufacture of a medicament) for use in the treatment of a genetic disorder or epigenetic disorder which are selected from or manifests itself in symptoms or diseases selected from urea cycle disorders, thalassemia, cystic fibrosis, rheumatoid arthritis, Siogren' s syndrome, uveitis, polymyositis, and dermatomyositis, arteriosclerosis, amyotrophic lateral sclerosis, asociality, affective disorders, systemic lupus erythematosus, immune response, varicosis, vaginitis, including chronic recurrent yeast vaginitis, depression or Sudden Infant Death Syndrome; especially in the treatment of cardiovascular disease like those being selected from arteriosclerosis, atherosclerosis, coronary heart disease, coronary artery disease, ischemic heart disease, angina, heart attack, heart failure, stroke and hypertension; or cystic fibrosis; or immune disease or autoimmune disease, like those being selected from rheumatoid arthritis, psoriatic arthritis, siogren's syndrome, uveitis, polymyositis, dermatomyositis, systemic lupus erythematosus, Crohn's disease, Graves' disease, Guillain-Barre syndrome, Hashimoto's thyroiditis, idiopathic thrombocytopenic purpura, psoriasis, ulcerative colitis, vasculitis and ankylosing spondylitis; or neurological or neurodegenerative diseases like those being selected from Rubinstein-Taybi syndrome, Rett syndrome, Friedreich's ataxia, Huntigton's disease, Parkinson's disease, amyotrophic lateral sclerosis and depression, or in the treatment of Alzheimer's Disease.

In a further aspect, the invention also refers to a method of treating a patient suffering from any of the above mentioned diseases by applying a suitable/physiologically effective amount of the pharmaceutical composition according to the invention.

The daily dosage for humans and animals may vary depending on factors that have their basis in the respective species or other factors, such as age, sex, weight or degree of illness and so forth. The daily dosage for humans preferably is in the range of 10 to 2000 milligrams of active substance (joined mixture according to the invention) to be administered during one or several intakes per day.

## Claims

1. A sustained release formulation comprising pellets containing 4-phenylbutyric acid (4-PBA), wherein the 4-PBA is in the form of the free acid.

2. A sustained release formulation according to claim 1, wherein the pellet is a homogenous pellet.

3. A sustained release formulation according to claim 1, wherein the pellet is a heterogeneous pellet.

4. A sustained release pellet according to claim 3, wherein the pellet core is an inert core, preferably comprising sugar or a cellulose derivative or a mixture thereof.

5. A sustained release formulation according to any of claims 1 to 4, wherein the pellet further comprises at least one pellet-forming substance preferably selected from the group consisting of microcrystalline cellulose, low-substituted hydroxypropyl cellulose, chitin, chitosan starch, polyvinylpyrrolidone, gelatine, hydroxypropylmethyl cellulose, hydroxyethyl cellulose, xanthan, polyvinyl acetate, sodium carboxymethyl cellulose, ethyl cellulose, liquefied waxes, or a mixture thereof.

6. A sustained release formulation according to any of the above claims, wherein the pellet further comprises at least one sedimentation retarder, preferably selected from the list consisting of highly disperse silicon dioxide, starch mucilage, mucilages like tragacanth, gums, e.g. gum arabic, xanthans, aliginates, carrageenan products, or a mixture thereof.

7. A sustained release formulation according to any of the above claims, wherein the pellet further comprises at least one pH corrector, preferably selected from the list comprising sodium hydroxide, hydrochloric acid, methylglucamine or buffer substances like sodium dihydrogen phosphate or disodium hydrogen phosphate.

8. A sustained release formulation according to any of the above claims, wherein the pellet further comprises at least one plasticizer, preferably selected from the group consisting of selected from the group consisting of polyethylene glycol, propylene glycol, glycerol, triacetin, dimethyl phthalate, diethyl phthalate, dibutyl phthalate, dibutyl sebacate, triethyl citrate, acetyltriethyl citrate, tributyl citrate, triethyl acetyl citrate, castor oil, poloxamer and varying percentages of acetylated monoglycerides or mixtures thereof.

9. A sustained release formulation according to any of the above claims, wherein the pellet further comprises at least one pigment, preferably selected from the group consisting of iron oxides, erythrosine, yellow orange S, tartraziane or indigotin.

10. A sustained release formulation according to any of the claims 3 to 9, wherein the pellet comprises a control release coating or wherein the pellet comprises a neutral film membrane.

11. A sustained release formulation according to any of the claims 3 to 9, wherein the pellet comprises an enteric coating.

12. A sustained release pellet according to any of the above claims, wherein the pellets are processed to a final dosage form which is selected from the group consisting of a tablet, sachet or capsule.

13. A process for the manufacture of a sustained release formulation according to any of the above claims wherein the process is selected from the list comprising moist granulation, melt granulation, dry granulation, globulation/droplet formation, cryopelletization, balling or compression.

14. A sustained release formulation according to any of claims 1 to 12 for use in therapy.

15. A sustained release formulation according to claim 14 the treatment of cancer, mucositis, amyotropic lateral sclerosis (ALS), skin/mucosal itching, degenerative diseases of the eye, neurodegenerative disorders, Huntington's disease, eating disorders, obesity, drug induced weight gain, pruritis, alcoholism, promyelocytic leukemia, especially acute promyelocytic leukemia, cardiac injury, especially adriamycin induced cardiac injury, epithelial damage, connective tissue damage, desmosis, Parkinson Disease, myelodysplastic syndrome, urea cycle disorder, fibrotic lung diseases, hepatic encephalopathies, thioredoxin mediated diseases, human papilloma virus (HPV) infections, autoimmune diseases, thalassemia, genetic disorders; or in the regulation of HDAC6 activity, the modulation of stem cells, the prevention of grey hair, the promotion of neuronal growth, the prevention of bone and joint diseases, or as adjuvant in cancer therapy or for the treatment of Alzheimer's Disease.
